# EUROPEAN PATENT APPLICATION

(11) **EP 3 098 297 A1**
(43) Date of publication of application: **30.11.2016**
(21) Application number: 15181334.2
(22) Date of filing: 18.08.2015
(51) Int. Cl.: C12M 1/12, C12M 1/42, C12M 1/00, C12M 3/06

(54) **A DUAL CHAMBER SELECTIVELY PERMEABLE DEVICE FOR CELL CULTURE AND THE APPLICATIONS THEREOF**

(30) Priority: 28.05.2015 CN 201510281271
(71) Applicant: Shenzhen Fulixin Health Industry Development Limited, Shenzhen, Hong Kong 51800 (CN); China Life Science Technology Group Limited, Wanchai, Hong Kong (CN)
(72) Inventor: LIU, Xiaoqing, 200072 Shanghai (CN)
(74) Representative: Heinze, Ekkehard

(57) **Abstract**

The present invention discloses a dual chamber selectively permeable cell culture device, wherein, the said device includes: at least two cell culture chambers: the first cell culture chamber and the second cell culture chamber; wherein, the said first cell culture chamber is configured to culture supporting cells, which are able to secrete supporting materials, and the said second cell culture chamber is applied to take the supporting materials and culture target cells; the said first cell culture chamber connects to the second cell culture chamber through a semipermeable membrane; each of the said first and second cell culture chamber has a liquid inlet and an inoculation channel. It is possible to reduce the cost for target cells culture effectively, while the structure of the said device is simple and easy to achieve, having a good market prospect.

## Description

### FIELD OF THE INVENTION

The present invention relates to the field of cell culture technologies, and more particularly, to a dual chamber selectively permeable device for cell culture and the applications thereof.

### BACKGROUND

Cell culture is a key technology widespread in the field of life science and regenerative medicine, adopting a regular basic culture medium (DMEM or RIPM1640) with some additional fetal bovine serum (FBS) may achieve conventional mammalian cells culture, however, for some special cells requiring the addition of many cytokines and other materials, this conventional cell culture system may create a pretty high cost for cell cultures. Thus, for this kind of special cells, reducing the cell culture cost becomes the key for sustainable development.

Following the rise of personalized cell therapy technology, *In vitro* proliferations of cells owning some special effects become a key study project in pre-clinical researches and clinical treatments. Those cells owning special functions after *in vitro* proliferations include multifunctional hematopoietic stem cells, T cells, NK cells, DC cells, CIK (cytokine-induced killer) cells and more. Transfusing back to mammal bodies after proliferating these cells *in vitro* may help to improve the functions of hematopoietic organs, such as: increasing the numbers of red blood cells (RBC) and immune cells, maintaining their functions, improving their anti-tumor capabilities, and their immune defense capabilities.

However, proliferating these cells with special functions *in vitro* need add a plurality of cytokines, proteins or antibodies, which may generate problems during culturing in a regular culture medium, including high costs and complex operations, while there are no technologies able to solve the above mentioned problems effectively in the current art.

Therefore, the prior art needs to be improved and developed.

### BRIEF SUMMARY OF THE DISCLOSURE

The technical problem to be solved in the present invention, aiming at the defects of the prior art, provides a dual chamber selectively permeable cell culture device and the applications thereof, in order to solve the problems in the prior art, that the cost is high and operations are complicated while adopting a conventional culture medium for those cells requiring additional supporting materials.

The technical solution of the present invention to solve the said technical problems is as follows:
A dual chamber selectively permeable device for cell culture, wherein, the said device includes: at least two chambers for cell culture: the first cell culture chamber and the second cell culture chamber; wherein, the said first cell culture chamber is configured to culture supporting cells, which are able to secrete supporting materials, and the said second cell culture chamber is applied to take the supporting materials and culture target cells; the said first cell culture chamber connects to the second cell culture chamber through a semi-permeable membrane; each of the said first cell culture chamber and the second cell culture chamber has a liquid inlet and an inoculation channel.

The said dual chamber selectively permeable cell culture device, wherein, the said cell culture chamber comprises culture dishes, culture plates or culture tanks, suitable for adherent cell cultures, culture tanks with multi-filters suitable for high-density cell cultures, and one kind of the culture bags or culture tanks suitable for suspension cell cultures.

The said dual chamber selectively permeable cell culture device, wherein, the said semi-permeable membrane has pore sizes no larger than 0.22 µm.

The said dual chamber selectively permeable cell culture device, wherein, the said supporting materials include one or more of cytokines, protein polypeptides, antibodies and other small molecule materials.

The said dual chamber selectively permeable cell culture device, wherein, the said cell culture chamber is fixed on a shakable device, applied to promote the exchange of non-cell components between cell chambers.

The said dual chamber selectively permeable cell culture device, wherein, the said first cell culture chamber and second cell culture chamber are placed horizontally, the said first cell culture chamber connects to the second cell culture chamber through a tubule with semi-permeable membranes.

The said dual chamber selectively permeable cell culture device, wherein, the said first cell culture chamber and second cell culture chamber are placed vertically, the bottom of the said first cell culture chamber connects to the top of the second cell culture chamber through semi-permeable membranes.

The said dual chamber selectively permeable cell culture device, wherein, the said first cell culture chamber contains multiple layers of filtration membranes for cell culture, applied to culture adhesive cells in a high density.

The said dual chamber selectively permeable cell culture device, wherein, a non-sealed cover is arranged on the top of the said first cell culture chamber, and a valve structure for solution exchanges is arranged at the bottom of the said second cell culture chamber.

An application of the said dual chamber selectively permeable cell culture device, wherein, the said dual chamber selectively permeable cell culture device is applied specifically to culture cells requiring the addition of supporting materials.

Benefits: the dual chamber selectively permeable device for cell culture and the applications thereof, as described in the present invention, includes at least two chambers for different cells culture: the first cell culture chamber and the second cell culture chamber, separated by a semi-permeable membrane, which makes small molecules including cytokines, antibodies and else, released by cells in the first cell culture chamber be able to enter the second cell culture chamber through the semi-permeable membranes before consumed by cells for culture there, also, cells cultured in both cell culture chambers will not get mixed due to passing through the semi-permeable membrane. It is possible to reduce the cost for target cells culture effectively, while the structure of the said device is simple and easy to achieve, having a good market prospect.

### BRIEF DESCRIPTION OF THE DRAWINGS

FIG. 1 illustrates the structure diagram of a dual bag-style-chamber cell culture device described in embodiment 1 in the present invention.
FIG. 2 illustrates the structure diagram of a dual tank-style-chamber cell culture device described in embodiment 2 in the present invention.
FIG. 3 illustrates the structure diagram of a dual tank-style-chamber cell culture device with multi-filters described in embodiment 3 in the present invention.
FIG. 4 illustrates a dual tank-style-chamber cell culture device owning solutions exchangeable functions described in embodiment 4 in the present invention.
FIG. 5 illustrates a dual-tank-style-chamber cell culture device with multi-filters owning solutions exchangeable functions described in embodiment 4 in the present invention.

### DETAILED DESCRIPTION

The present invention provides a dual chamber selectively permeable cell culture device and the applications thereof, in order to make the purpose, technical solutions and the advantages of the present invention clearer and more explicit, further detailed descriptions of the present invention are stated here, referencing to the attached drawings and some embodiments of the present invention. It should be understood that the detailed embodiments of the invention described here are used to explain the present invention only, instead of limiting the present invention.

The present invention provides a dual chamber selectively permeable device for cell culture, wherein, the said device includes: at least two chambers for different cells culture: the first cell culture chamber and the second cell culture chamber; wherein, the said first cell culture chamber is configured to culture supporting cells, which are able to secrete supporting materials, and the said second cell culture chamber is applied to take the supporting materials and culture target cells; the said first cell culture chamber connects to the second cell culture chamber through a semi-permeable membrane; each of the said first cell culture chamber and the second cell culture chamber has a liquid inlet and an inoculation channel.

The embodiment provided in the present invention, has two cell culture chambers separated by a semi-permeable membrane, and that makes the cytokines, antibodies and other small molecule materials released by cells in the first cell culture chamber enter the second cell culture chamber through the semi-permeable membrane in between before consumed by cells cultured in the second chamber, while cells cultured in both cell culture chambers will not get mixed due to not being able to pass through the semi-permeable membranes. It is possible to reduce the cost for target cells culture effectively, also the structure of the said device is simple and easy to achieve, having a good market prospect.

Specifically, each cell culture chamber cultures different cells separately, wherein, the first cell culture chamber cultures supporting cells which may provide multiple supporting materials including cytokines, protein polypeptides and antibodies, and these supporting materials are required for those target cells cultured in the second cell culture chamber. By configuring two cell culture chambers for different cells culture, the producing cost for those target cells that need extra supporting materials, may get reduced.

Additionally, the said first cell culture chamber connects to the second cell culture chamber through a semi-permeable membrane. A semi-permeable membrane is a selectively permeable membrane, allowing only ions and small molecules to pass through freely, while those biomacromolecules such as cells and else failed to pass through freely, the reason is, the pore sizes of a semi-permeable membrane are larger than the sizes of ions and small molecules, while they are smaller than that of biomacromolecules including proteins and starch. The present invention adopts semi-permeable membranes to connect the first cell culture chamber and the second cell culture chamber, making those small molecules be exchanged freely in between the two chambers, for example, the cytokines, protein polypeptides and other small molecules, excreted by the supporting cells cultured in the first cell culture chamber, may pass through the semi-permeable membrane and enter the second cell culture chamber, taken by target cells for growth, they may promote cell proliferations or promote some kind of activations to target cells, while both the supporting cells and target cells cultured in the two cell culture chambers separately won't get exchanged through the semi-permeable membranes, that keeps the unity of each cell culture effectively, and avoids cells getting mixed up.

In a preferred embodiment, the said cell culture chamber comprises culture dishes, culture plates, culture tanks, culture tanks with multi-layers of filtration membranes suitable for high-density cell cultures and one type of the culture bags or culture tanks suitable for suspension cell cultures. It is possible to choose different culture containers to be the cell culture chambers for the said dual chamber selectively permeable cell culture device as described in the present invention, and arrange a liquid inlet and an inoculation channel into the said cell culture chamber, applied to add the nutrition solutions and inoculate cells, and achieve the cell cultures. Additionally, the cell culture chambers described in the present invention allow gases such as CO₂ to enter or exit freely. Furthermore, the said liquid inlet and inoculation channel may be arranged on the upper positions of the said cell culture chamber. It should be noted that, high-density cell culture, as described in the present invention, means the cells are cultured in a density close to the highest theoretical value. Due to the big differences in cell types and target products, the final cell numbers in a high-density cell culture is impossible to define with an exact value or range. For example, when the cell concentration of the engineering bacteria in a broth is above 50gDCW/L, it is possible to become a high-density fermentation for E. coli, while for some extreme microbial, several grams per liter in cell density is enough to be considered a high-density culture.

Preferably, the said semi-permeable membrane has pore sizes no larger than 0.22 µm. Specifically, the said semi-permeable membrane has a pore size of 0.22 µm or 0.1 µm, of course, it is also possible to choose semi-permeable membranes with some other pore sizes, based on the actual needs in productions. The semi-permeable membranes described in the present invention allow cytokines, protein polypeptides, antibodies or other small molecule materials to pass through freely, instead of the cultured supporting cells or target cells passing through, thus avoid different cells mixing up.

When the first cell culture chamber and the second cell culture chamber are placed vertically, a non-sealed cover is arranged on top of the first cell culture chamber, and a valve structure applied for solutions exchanges is arranged on the bottom of the second cell culture chamber. Specifically, the said non-sealed cover may be made by a plurality of materials including plastics, and it covers the top of the first cell culture chamber, avoiding pollutions including dusts entering the cell culture chamber effectively, while adopting a non-sealed cover, allows gases in the cell culture chamber (such as CO₂) to exchange with gases outside. Additionally, the said valve structure may be arranged in the plastic tubule connecting to the bottom of the second cell culture chamber, of course, a semi-permeable membrane structure is also arranged at the connection position where the said plastic tubule connects to the bottom of the second cell culture chamber, to avoid cells lost. When culturing cells, the valve structure is closed, and when exchanging solutions, the valve structure is open, and the plastic tubule connects to a vacuum through a hose, removing some old culture medium through vacuuming, and new culture medium is added through liquid inlet and inoculation channel.

The present invention also provides an implementation on the above said dual chamber selectively permeable cell culture device, wherein, the said dual channel selectively permeable cell culture device is specifically applied to culture those cells need extra supporting materials; the said supporting materials include one or more of cytokines, protein polypeptides, antibodies and other small molecule materials. More pacifically, the said supporting materials include one or more of fibroblast growth factor 1, fibroblast growth factor 2, epidermal growth factor, platelet-derived growth factor, insulin, insulin-like growth factor 1, vascular endothelial growth factor, placental growth factor, leukemia inhibitory factor, transferrin, colony stimulating factor 1, tumor necrosis factor, tumor transforming factor, macrophage colony stimulating factor, granulocyte colony-stimulating factor, interleukin-2, interleukin-4, interleukin-5, interleukin-6, interleukin-12, interleukin-15, interleukin-23, interferon, erythropoietin, thrombopoietin, stem cell factors, anti-CD3 antibodies and anti-CD28 antibodies.

The following embodiments further describe the technical solution of the present invention.

### Embodiment I:

A dual bag-style-chamber cell culture device

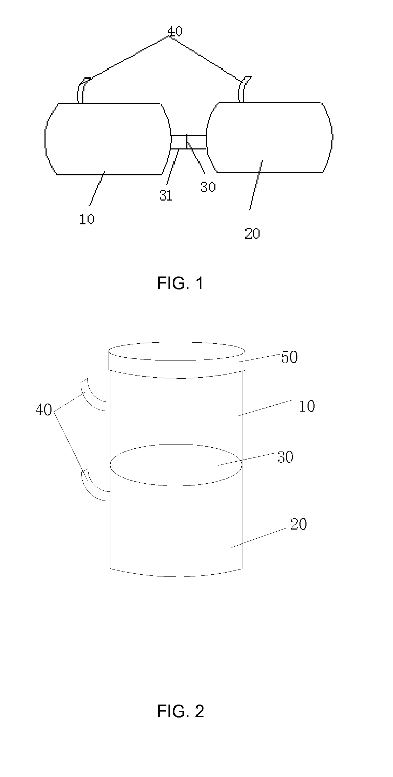

A dual bag-style-chamber cell culture device described in the present embodiment has the following characters: the material used for the cell culture bags allows gases (such as CO₂) to pass freely, but not other liquid molecules to leak out. A tiny tubule connects the two culture bags together in between. A selectively semi-permeable membrane 30 locates in the middle of the connected tiny tubule, whose pore size is 0.22µm. This selectively semi-permeable membrane 30 allows those cytokines, protein polypeptides, antibodies and other small molecule materials excreted by cells cultured in the first cell culture chamber 10 to reach the second cell culture chamber 20, and consumed by cells cultured there. These cytokines, protein polypeptides, antibodies and other small molecule materials may promote cell proliferations or promote some kind of activations to cells cultured in the second cell culture chamber 20, each culture bag has its corresponding cell inoculation and refilling inlets 40 (see FIG. 1), also, the said culture bag may be placed on a mildly rotating or vibrating apparatus, since this kind of rotation or vibration may promote different components in the broth, other than the cells, to get exchanged thoroughly.

### Embodiment II:

A dual tank-style-chamber cell culture device

A dual tank-style-chamber cell culture device described in the present embodiment has the following characters: the two cell culture tanks are placed vertically in the space, and the selectively semi-permeable membrane 30 connected in the middle has a pore size of 0.1µm. This kind of selectively semi-permeable membrane 30 allows those cytokines, protein polypeptides, antibodies and other small molecule materials excreted by cells cultured in the first cell culture chamber 10 to reach the second cell culture chamber 20, and consumed by cells cultured there. These cytokines, protein polypeptides, antibodies and other small molecule materials passed through the selectively semi-permeable membrane may promote cell proliferations or promote some kind of activations to cells cultured in the second cell culture chamber 20. The first cell culture chamber 10 is covered by a cover 50 without getting fully sealed, allowing gases (such as CO2) to exchange with outside (see FIG. 2). This kind of device may be placed on a mildly rotating or vibrating apparatus, since this kind of rotation or vibration may promote different components in the broth, other than the cells, to get exchanged thoroughly.

### Embodiment III

A dual tank-style-chamber cell culture device with multi-filters

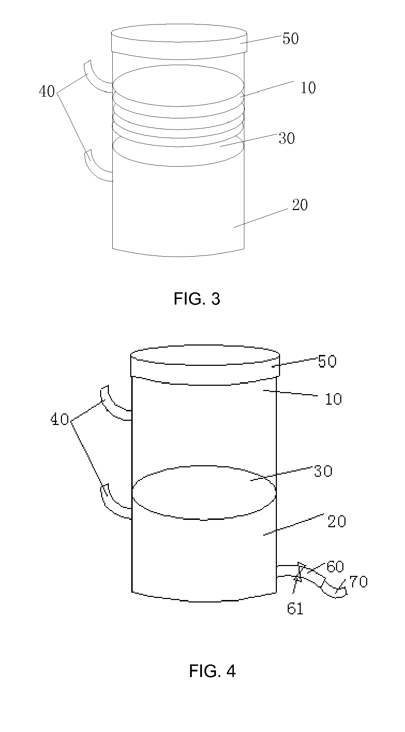

The dual tank-style-chamber cell culture device with multi-filters, described in the present embodiment, is improved based on that in the embodiment II, and the specific improvements are listed below: multilayer of cell culture filters 30 (i.e., multilayer of selectively semi-permeable membranes) are placed vertically in the first cell culture chamber 10, and are able to culture adhesive cells in a high density, while the pore size of the selectively semi-permeable membranes is 0.16µm. The selectively semi-permeable membrane not only allows the cells to grow adhesively, also allows those cytokines, protein polypeptides, antibodies and other small molecule materials excreted by cells cultured in the first cell culture chamber 10 to reach the second cell culture chamber 20, and consumed by the cells cultured in the second cell culture chamber 20. These cytokines, protein polypeptides, antibodies and other small molecule materials passed through the selectively semi-permeable membrane may promote cell proliferations or promote some kind of activations to cells cultured in the second cell culture chamber 20 (see FIG. 3). This kind of device may be placed on a mildly rotating or vibrating apparatus, since this kind of rotation or vibration may promote different components in the broth, other than the cells, to get exchanged thoroughly.

### Embodiment IV

A solution exchangeable dual tank-style-chamber cell culture device and a solution exchangeable dual tank-style-chamber cell culture device with multi-filters

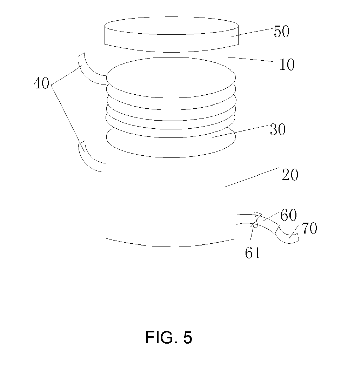

The solution exchangeable dual tank-style-chamber cell culture device, described in the present embodiment, is improved based on that in the embodiment II (or embodiment III). The specific improvements are listed below: a plastic catheter 60 is connected to the bottom of the second cell culture chamber 20, and a switchable valve 61 is arranged on the plastic catheter 60, which is closed during cell culturing; and gets open in solution exchanging during culturing. The plastic catheter 60 connects to a vacuum through a latex tube 70, removing the old medium when the vacuum is on, and adding new culture medium through both liquid inlet and inoculation channel (see FIG. 4). The first cell culture chamber may also be designed with multi-filters, to achieve the adhesive cells culture in a high density (see FIG. 5). This kind of apparatus may be placed on a mildly rotating or vibrating apparatus, since this kind of rotation or vibration may promote different components in the broth, other than the cells, to get exchanged thoroughly.

All together, the dual chamber selectively permeable device for cell culture and the applications thereof, as described in the present invention, includes at least two chambers for different cells culture: the first cell culture chamber and the second cell culture chamber, separated by a semi-permeable membrane, which makes small molecules including cytokines, antibodies and more released by cells in the first cell culture chamber be able to enter the second cell culture chamber through the semi-permeable membrane before consumed by cells for culture there, also, cells cultured in either cell culture chamber will not get mixed due to passing through the semi-permeable membrane. It is possible to reduce the cost for target cells culture effectively, while the structure of the said device is simple and easy to achieve, having a good market prospect.

It should be understood that, the application of the present invention is not limited to the above examples listed. It will be possible for a person skilled in the art to make modifications or replacements according to the above descriptions, which shall all fall within the protection scope of the appended claims of the present invention.

## Claims

1. A dual chamber selectively permeable device for cell culture, wherein, the said device includes: at least two cell culture chambers: the first cell culture chamber and the second cell culture chamber; wherein, the said first cell culture chamber is configured to culture supporting cells, which are able to secrete supporting materials, and the said second cell culture chamber is applied to take the supporting materials and culture target cells;
the said first cell culture chamber connects to the second cell culture chamber through a semi-permeable membrane; each of the said first cell culture chamber and the second cell culture chamber has a liquid inlet and an inoculation channel.

2. The said dual chamber selectively permeable cell culture device according to claim 1, wherein, the said cell culture chamber comprises culture dishes, culture plates or culture tanks, suitable for adherent cell cultures, culture tanks with multi-filters suitable for high-density cell cultures, and one kind of the culture bags or culture tanks suitable for suspension cell cultures.

3. The said dual chamber selectively permeable cell culture device according to claim 1, wherein, the said semi-permeable membrane has pore sizes no larger than 0.22 µm.

4. The said dual chamber selectively permeable cell culture device according to claim 1, wherein, the said supporting materials include one or more of cytokines, protein polypeptides, antibodies and other small molecule materials.

5. The said dual chamber selectively permeable cell culture device according to claim 1, wherein, the said cell culture chamber is fixed on a shakable device, applied to promote the exchange of non-cell components between cell chambers.

6. The said dual chamber selectively permeable cell culture device according to claim 1, wherein, the said first cell culture chamber and second cell culture chamber are placed horizontally, the said first cell culture chamber connects to the second cell culture chamber through a tubule with semi-permeable membranes.

7. The said dual chamber selectively permeable cell culture device according to claim 1, wherein, the said first cell culture chamber and second cell culture chamber are placed vertically, the bottom of the said first cell culture chamber connects to the top of the second cell culture chamber through semi-permeable membranes.

8. The said dual chamber selectively permeable cell culture device according to claim 7, wherein, the said first cell culture chamber contains multiple layers of filtration membranes for cell culture, applied to culture adhesive cells in a high density.

9. The said dual chamber selectively permeable cell culture device according to claim 7, wherein, a non-sealed cover is configured on the top of the said first cell culture chamber, and a valve structure for solution exchange is arranged at the bottom of the said second cell culture chamber.

10. An application of the said dual chamber selectively permeable cell culture device according to anyone in claims 1 to 9, wherein, the said dual chamber selectively permeable cell culture device is applied specifically to culture cells requiring the addition of supporting materials.
